(19) Europäisches Patentamt
European Patent Office
Office européen des brevets

(11) **EP 3 401 297 A1**

(12) **EUROPEAN PATENT APPLICATION**
published in accordance with Art. 153(4) EPC

(43) Date of publication:
**14.11.2018 Bulletin 2018/46**

(51) Int Cl.:
*C07C 17/383* [(2006.01)]    *C07C 17/25* [(2006.01)]
*C07C 21/18* [(2006.01)]    *C07B 61/00* [(2006.01)]

(21) Application number: **16883772.2**

(22) Date of filing: **19.12.2016**

(86) International application number:
**PCT/JP2016/087804**

(87) International publication number:
**WO 2017/119273 (13.07.2017 Gazette 2017/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **06.01.2016 JP 2016001037**

(71) Applicant: **Daikin Industries, Ltd.**
**Osaka 530-8323 (JP)**

(72) Inventors:
• **TAKAKUWA, Tatsuya**
**Osaka-shi**
**Osaka 530-8323 (JP)**

• **TAKAHASHI, Kazuhiro**
**Osaka-shi**
**Osaka 530-8323 (JP)**
• **KARUBE, Daisuke**
**Osaka-shi**
**Osaka 530-8323 (JP)**
• **CHAKI, Takehiro**
**Osaka-shi**
**Osaka 530-8323 (JP)**
• **KISHIMOTO, Masayuki**
**Osaka-shi**
**Osaka 530-8323 (JP)**
• **KOMATSU, Yuzo**
**Osaka-shi**
**Osaka 530-8323 (JP)**

(74) Representative: **Hoffmann Eitle**
**Patent- und Rechtsanwälte PartmbB**
**Arabellastraße 30**
**81925 München (DE)**

(54) **METHOD FOR PRODUCING 2, 3, 3, 3-TETRAFLUOROPROPENE**

(57)    This invention provides a method for stably producing 2,3,3,3-tetrafluoropropene for a long period of time while suppressing catalyst deactivation. This invention provides a method for producing 2,3,3,3-tetrafluoropropene, the method comprising: (d) reacting 2-chloro-3,3,3-trifluoropropene with hydrogen fluoride in the presence of a catalyst; (e) subjecting the reaction mixture obtained in step (d) to distillation to separate the mixture into a first stream comprising 2,3,3,3-tetrafluoropropene as a main component and a second stream comprising unreacted hydrogen fluoride and organic matter containing unreacted 2-chloro-3,3,3-trifluoropropene as main components; and (f) recycling the second stream separated in step (e) above to the reaction of step (d), wherein the distillation of step (e) is performed under conditions that satisfy the relationship of a specific equation.

EP 3 401 297 A1

Fig. 1

Starting
material

HF

10a  1a  2a  20a

11a

Fractional distillation

21a

10b  2b  20b
1b

11b

Fractional distillation

HF

21b

2

**Description**

Technical Field

The present invention relates to a method for producing 2,3,3,3-tetrafluoropropene.

Background Art

Alternative refrigerants, such as HFC-125($C_2HF_5$) and HFC-32($CH_2F_2$), have been widely used as important replacements for CFC, HCFC, etc., which cause ozone layer depletion. However, these alternative refrigerants are potent global warming substances, thus creating concern that diffusion of the refrigerants would increase global warming. As a preventive measure, these refrigerants are recovered after use. However, complete recovery of the refrigerants is impossible. In addition, diffusion of these refrigerants due to, for example, leakage, cannot be ignored. The use of $CO_2$ or hydrocarbon-based substances as alternative refrigerants has also been investigated. However, because $CO_2$ refrigerants have low efficiency, and devices using such refrigerants inevitably become large, $CO_2$ refrigerants have many problems in terms of the overall reduction of greenhouse gas emissions, including energy to be consumed. Furthermore, hydrocarbon-based substances pose safety problems due to their high flammability.

HFO-1234yf ($CF_3CF=CH_2$), which is an olefinic HFC with low global warming potential, has recently been attracting attention as a material to solve the above problems. HFO-1234yf, used alone or in combination with other substances, such as hydrofluorocarbons (HFCs), hydrofluoroolefins (HFOs), and hydrochlorofluoroolefins (HCFOs), is expected to be useful as a refrigerant, and additionally as a blowing agent, propellant, extinguishing agent, and the like.

Various methods are known for producing HFO-1234yf. For example, there have been proposed methods such as a method in which $CCl_3CF_2CH_3$ as a starting material is reacted with hydrogen fluoride (HF) that has an amount exceeding the stoichiometric amount (Patent Literature 1), and a method in which a fluorocarbon represented by $CF_3CFHCFH_2$ is subjected to dehydrofluorination treatment (Patent Literature 2).

Citation List

Patent Literature

PTL 1: US Patent Application Publication No. 2996555
PTL 2: WO2008/002499

Summary of Invention

Technical Problem

In the production methods disclosed in the Patent Literature mentioned above, the conversion of HCFO-1233xf to HFO-1234yf is as low as 20% or less. Additionally, the outflow from the reactor contains not only the desired product HFO-1234yf, but also a mixture containing the unreacted HCFO-1233xf and HF in an amount at least equimolar to that of the unreacted HCFO-1233xf. Thus, by distillation treatment, the desired HFO-1234yf is withdrawn from the top of the distillation column, and other components, i.e., HF and HCFO-1233xf, are withdrawn from the bottom of the distillation column and recycled by feeding HF and HCFO-1233xf again to the reactor. However, depending on the molar ratio of HF and HCFO-1233xf or the still temperature, HF and HCFO-1233xf may undergo liquid-liquid separation in the still. As a result, a high concentration of an organic phase at the lower phase is fed to the reactor. Such a high concentration of the organic phase fed to the reactor poses a problem of deactivation of the catalyst caused by the action of the organic matter.

The present invention has been accomplished in view of the above. An object of the present invention is to provide a method for stably producing 2,3,3,3-tetrafluoropropene for a long period of time in which unreacted materials are reused after distillation without liquid-liquid separation to suppress catalyst deactivation. Another object of the present invention is to provide a method for stably producing a chloropropene used for the production of 2,3,3,3-tetrafluoropropene for a long period of time in which unreacted materials are reused after distillation without liquid-liquid separation to suppress catalyst deactivation, as in the method for producing 2,3,3,3-tetrafluoropropene.

Solution to Problem

The present inventors conducted extensive research to achieve the above objects and found that the objects can be achieved by performing distillation under conditions in which unreacted materials including hydrogen fluoride and organic matter such as HCFO-1233xf as main components do not undergo liquid-liquid separation. The present invention has thus been accomplished.

Specifically, the present invention relates to the following method for producing a chloropropene and the following method for producing 2,3,3,3-tetrafluoropropene.

1. A method for producing 2,3,3,3-tetrafluoropropene from 2-chloro-3,3,3-trifluoropropene, the method comprising the following steps (d) to (f):

(d) reacting 2-chloro-3,3,3-trifluoropropene with hydrogen fluoride in the presence of a catalyst;
(e) subjecting the reaction mixture obtained in step (d) to distillation to separate the mixture into a first stream comprising 2,3,3,3-tetrafluoropropene as a main component and a second stream comprising unreacted hydrogen fluoride and organic matter containing unreacted 2-chloro-3,3,3-trifluoropropene as main components; and
(f) recycling the second stream separated in step (e) above to the reaction of step (d),

wherein the distillation of step (e) is performed under conditions that satisfy the relationship of the following equation (1):

$$Y_2 \geq -0.000027546483936X_2^5 + 0.001787977446337X_2^4$$
$$-0.046613778365698X_2^3 + 0.617838436698327X_2^2$$
$$-4.260415044902270X_2 + 12.671429439720600 \quad (1),$$

wherein $Y_2$ is the pressure in a distillation column where the distillation of step (e) is performed, and $X_2$ is the molar ratio of the hydrogen fluoride to the organic matter in the distillation column.

2. The production method according to Item 1, wherein the molar ratio $X_2$ is 10 or more, and the pressure $Y_2$ is 0 MPa or more but 1 MPa or less.

3. A method for producing 2,3,3,3-tetrafluoropropene, the method comprising:

a first stage of obtaining 2-chloro-3,3,3-trifluoropropene from a starting material containing a chloropropane represented by formula (Ia): $CX_3CClYCH_2Y$, wherein X is Cl or F, each X may be the same or different, Y is H, F, or Cl, and each Y may be the same or different, and/or a chloropropene represented by formula (Ib): $CY_3CCl=CZ_2$, wherein Y is H or Cl, each Y may be the same or different, Z is H, F, or Cl, and each Z may be the same or different; and
a second stage of obtaining 2,3,3,3-tetrafluoropropene from the 2-chloro-3,3,3-trifluoropropene,
the first stage comprising the following steps (a) to (c);

(a) reacting the starting material with hydrogen fluoride in the presence of a catalyst;
(b) subjecting the reaction mixture obtained in step (a) to distillation to separate the mixture into a first stream comprising the 2-chloro-3,3,3-trifluoropropene as a main component and a second stream comprising an unreacted starting material and unreacted hydrogen fluoride as main components;
(c) recycling the second stream separated in step (b) to the reaction of step (a),

the second stage comprising the following steps (d) to (f):

(d) reacting the 2-chloro-3,3,3-trifluoropropene with hydrogen fluoride in the presence of a catalyst;
(e) subjecting the reaction mixture obtained in step (d) to distillation to separate the mixture into a first stream comprising 2,3,3,3-tetrafluoropropene as a main component and a second stream comprising unreacted hydrogen fluoride and organic matter containing unreacted 2-chloro-3,3,3-trifluoropropene as main components; and
(f) recycling the second stream separated in step (e) to the reaction of step (d),

wherein the distillation of step (e) is performed under conditions that satisfy the relationship of the following equation (1):

$$Y_2 \geq -0.000027546483936X_2^5 + 0.001787977446337X_2^4$$
$$-0.046613778365698X_2^3 + 0.617838436698327X_2^2$$
$$-4.260415044902270X_2 + 12.671429439720600 \quad (1),$$

wherein $Y_2$ is the pressure in a distillation column where the distillation of step (e) is performed, and $X_2$ is the molar ratio of the hydrogen fluoride to the organic matter in the distillation column.

4. The production method according to Item 3, wherein the distillation of step (b) is performed under conditions in which the unreacted starting material and the unreacted hydrogen fluoride do not undergo liquid-liquid separation.

5. The production method according to Item 3 or 4, wherein the molar ratio $X_2$ is 10 or more, and the pressure $Y_2$ is 0 MPa or more but 1 MPa or less.

6. The production method according to any one of Items 3 to 5, wherein the starting material is 1,1,1,2,3-pentachloropropane, the amount of the hydrogen fluoride supplied to a reactor is 15 moles or more, per mole of the starting material supplied to the reactor, and the pressure in the distillation column where the distillation of step (b) is performed is 0 MPa or more but 1 MPa or less.

Advantageous Effects of Invention

In the production of a chloropropene in the present invention, distillation is performed under conditions in which a mixture containing unreacted starting material and unreacted hydrogen fluoride that remain after a reaction of the starting material does not undergo liquid-liquid separation, and a fraction obtained by the distillation is reused in the reaction. Thus, catalyst deactivation in the reaction system can be suppressed, enabling the chloropropene to be stably produced for a long period of time.

Moreover, in the production of 2,3,3,3-tetrafluoropropene in the present invention, distillation is performed under conditions in which a mixture containing unreacted raw material and unreacted hydrogen fluoride that remain after a reaction of the raw material does not undergo liquid-liquid separation, and a fraction obtained by the distillation is reused in the reaction. Thus, catalyst deactivation in the reaction system can be suppressed, enabling 2,3,3,3-tetrafluoropropene to be stably produced for a long period of time.

Brief Description of Drawings

Fig. 1 is a flow diagram illustrating an example of the method for producing 2,3,3,3-tetrafluoropropene.
Fig. 2 is a liquid-liquid separation curve plotting the pressure in the distillation column versus the hydrogen fluoride/chloropropene intermediate molar ratio.
Fig. 3 is a flow diagram illustrating an example of the method for producing 2,3,3,3-tetrafluoropropene and is a schematic flow diagram illustrating the recycling process from the distillation column to the reactor.
Fig. 4 is a graph plotting the conversion of 1233xf versus the reaction time.
Fig. 5 is a graph plotting the reaction yield of 1234yf versus the reaction time.

Description of Embodiments

Embodiments of the present invention are described in detail below.

Method for Producing Chloropropene

The chloropropene is represented by formula (II): $CX_3CCl=CH_2$, wherein at least one X is F and the other or others are Cl or F, and each X may be the same or different. This chloropropene is produced from a starting material containing a chloropropane represented by formula (Ia): $CX_3CClYCH_2Y$, wherein X is Cl or F and each X may be the same or different, Y is H, F, or Cl and each Y may be the same or different, and/or a chloropropene represented by

formula (Ib): $CY_3CCl=CZ_2$, wherein Y is H or Cl and each Y may be the same or different, Z is H, F, or Cl and each Z may be the same or different.

Hereinafter, the chloropropane represented by formula (Ia) may be referred to as "starting material chloropropane," the chloropropene represented by formula (Ib) may be referred to as "starting material chloropropene," and the chloropropene represented by formula (II) may be referred to as "chloropropene intermediate." "Starting material chloropropane" and "starting material chloropropene" may be collectively referred to as "starting material." "Starting material" means either "starting material chloropropane" or "starting material chloropropene," or both.

Hereinafter, the stage of producing a chloropropene represented by formula (II) (chloropropene intermediate) from the starting material mentioned above is referred to as "first stage."

Specific examples of the starting material chloropropane of formula (Ia) include $CCl_3CHClCH_2Cl$ (which hereinafter may be referred to as "240db"), $CF_3CHClCH_2Cl$ (which hereinafter may be referred to as "243db"), $CF_3CClFCH_3$ (which hereinafter may be referred to as "244bb"), $CF_3CHClCH_2F$ (which hereinafter may be referred to as "244db"), $CFCl_2CHClCH_2Cl$ (which hereinafter may be referred to as "241db"), $CF_2ClCHClCH_2Cl$ (which hereinafter may be referred to as "242dc"), and the like. Among these, 240db (1,1,1,2,3-pentachloropropane), 243db (2,3-dichloro-1,1,1-trifluoropropane), and 244bb (2-chloro-1,1,1,2-tetrafluoropropane) are particularly preferable. The starting material chloropropanes may be used singly or in a combination of two or more.

Specific examples of the starting material chloropropene of formula (Ib) include 1,1,2,3-tetrachloropropene ($CCl_2=CClCH_2Cl$, which hereinafter may be referred to as "1230xa") and 2,3,3,3-tetrachloropropene ($CH_2=CClCCl_3$, which hereinafter may be referred to as "1230xf").

Specific examples of the chloropropene intermediate of formula (II) include 2-chloro-3,3,3-trifluoropropene ($CF_3CCl=CH_2$, which hereinafter may be referred to as "HCFO-1233xf" or "1233xf"), 1,2-dichloro-1,1-difluoro-3-propene ($CClF_2CCl=CH_2$, which hereinafter may be referred to as "1232xf"), and 1,1,2-trichloro-1-fluoro-3-propene ($CCl_2FCCl=CH_2$, which hereinafter may be referred to as "1231xf"). In the present invention, the chloropropene intermediate of formula (II) is preferably 2-chloro-3,3,3-trifluoropropene (HCFO-1233xf).

The first stage comprises the following steps (a) to (c):

(a) reacting the starting material with hydrogen fluoride in the presence of a catalyst;
(b) subjecting the reaction mixture obtained in step (a) to distillation to separate the mixture into a first stream comprising the chloropropene of formula (II) as a main component and a second stream comprising the unreacted starting material and the unreacted hydrogen fluoride as main components; and
(c) recycling the second stream separated in step (b) to the reaction of step (a).

In particular, in the present invention, the distillation of step (b) is performed under conditions in which the unreacted starting material and the unreacted hydrogen fluoride do not undergo liquid-liquid separation at a portion of the distillation column from which the second stream is withdrawn.

In step (a), the starting material is reacted with hydrogen fluoride to obtain a product containing a chloropropene intermediate. The product containing a chloropropene intermediate is a compound that serves as an intermediate in the production of 2,3,3,3-tetrafluoropropene.

The method for reacting the starting material with hydrogen fluoride in the presence of a catalyst is not particularly limited. Examples of specific embodiments of the method include a method in which a catalyst is placed in a tubular flow reactor, and the starting material and hydrogen fluoride are introduced into the reactor.

The starting material can be reacted with hydrogen fluoride in a gas phase. The starting material and hydrogen fluoride are brought into contact with each other in a gaseous state in the reaction temperature region described below. When the starting material is liquid at an ordinary temperature and ordinary pressure, the starting material may be evaporated using an evaporator and supplied to a reactor where the reaction of step (a) is performed.

Hydrogen fluoride may generally be supplied to a reactor in a gas phase together with the starting material. The amount of hydrogen fluoride supplied to a reactor is generally about 1 to 100 moles, preferably about 5 to 50 moles, and more preferably about 15 to 40 moles, per mole of the starting material. By setting the amount within such a range, the conversion of the starting material can be maintained within a desirable range. It is particularly preferable that hydrogen fluoride be supplied to a reactor in an amount of 15 moles or more, per mole of the starting material, because deactivation of the catalyst can be further suppressed.

The molar ratio of hydrogen fluoride and the starting material supplied to the reactor can be adjusted by the amounts of hydrogen fluoride and the starting material supplied to the reactor. Thus, regarding a stream not for recycling but for supplying the major raw materials to the reactor and the second stream, the flow rates of hydrogen fluoride and the starting material can be adjusted by additionally supplying hydrogen fluoride and the starting material or withdrawing them from the reactor.

Hydrogen fluoride and the starting material may be supplied to the reactor together with gas that is inert to the raw materials and the catalyst, such as nitrogen, helium, or argon. The concentration of inert gas may be about 0 to 10 mol% based on the total amount of the raw materials including the starting material and hydrogen fluoride introduced into the reactor and the inert gas, plus, when added, oxygen gas described later.

The starting material may be supplied to the reactor of step (a) together with oxygen or chlorine. In this case, the amount of oxygen or chlorine supplied may be about 0.1 to 50 mol% based on the total amount of the raw materials and oxygen, plus, when added, inert gas, or based on the total amount of the raw materials and chlorine, plus, when added, inert gas.

As a catalyst, known materials that have been used for this reaction can be used, and the type of catalyst is not particularly limited. For example, known catalysts usable in the dehydrohalogenation reaction can be used. Examples thereof include halides and oxides of transition metals, Group 14 and 15 elements of the Periodic Table, etc. Specific examples of transition elements include Ti, V, Cr, Mn, Fe, Co, Ni, Cu, Zn, Zr, Nb, Ta, W, and the like. Specific examples of Group 14 elements of the Periodic Table include Sn, Pb, and the like. Specific examples of Group 15 elements of the Periodic Table include Sb, Bi, and the like. Examples of halides of these elements include fluoride, chloride, and the like. These catalysts may be used singly or in a combination of two or more, and may be supported on a carrier. Examples of carriers include, but are not particularly limited to, porous alumina silicate typified by zeolite, aluminum oxide, silicon oxide, activated carbon, titanium oxide, zirconia oxide, zinc oxide, aluminum fluoride, and the like. These may be used singly or in a combination of two or more, or a structural composite form thereof.

The reactor is preferably a tubular reactor. The method for bringing a material into contact with the catalyst is preferably a method using a fixed layer. The reactor is preferably made of a material resistant to the corrosive action of hydrogen fluoride, such as Hastelloy (registered trademark), Inconel (registered trademark), or Monel (registered trademark).

In the reaction of step (a), the reaction temperature is not particularly limited and is generally preferably about 200°C to 550°C. When the temperature is in this range, excellent conversion of the starting material is exhibited, and the production of by-products caused by decomposition of the raw materials can be suppressed. The reaction temperature is more preferably about 300°C to 450°C.

The pressure during the reaction of step (a) is not particularly limited, and the reaction may be performed under reduced pressure, ordinary pressure, or increased pressure. Although the reaction may be generally carried out at a pressure near atmospheric pressure (0.1 MPa), it can also proceed smoothly under reduced pressure of less than 0.1 MPa. Further, the reaction may be performed under increased pressure within a range in which the raw materials do not liquefy.

There is no limitation on the reaction time. For example, the contact time represented by W/F0, i.e., the ratio of the amount of packed catalyst W(g) to the total flow rate F0 (a flow rate at 0°C and 0.1 MPa: cc/sec) of gas components supplied to the reaction system is preferably about 0.1 to 90 g·sec/cc, and more preferably about 1 to 50 g·sec/cc. In this case, the total flow rate of gas components means the total flow rate of the starting material and hydrogen fluoride, and, when used, inert gas, oxygen, etc.

A reaction mixture containing a chloropropene intermediate, which is a product, as a main component and also containing the unreacted starting material and the unreacted hydrogen fluoride is obtained by performing step (a) described above.

In step (b), the reaction mixture obtained in step (a) is subjected to distillation. By this distillation, the mixture can be separated into a first stream comprising the chloropropene intermediate as a main component and a second stream comprising the unreacted starting material and the hydrogen fluoride as main components. The portion of the distillation column from which the second stream is withdrawn may be the still of the distillation column or a middle portion of the distillation column. The distillation can be performed using a commonly used distillation column or the like.

The distillation is performed under conditions in which the unreacted starting material and unreacted hydrogen fluoride that form the second stream do not undergo liquid-liquid separation. The liquid-liquid separation as used here refers to a state in which two or more liquids (for example, a liquid phase of chloropropane and a liquid phase of hydrogen fluoride) undergo separation into two phases, and does not refer to a state in which two or more liquids are mixed as a single liquid phase.

Examples of the method for performing distillation under conditions in which liquid-liquid separation does not occur include a method in which the molar ratio of the hydrogen fluoride to the unreacted starting material in the distillation column, i.e., [the number of moles of the hydrogen fluoride]/[the number of moles of the unreacted starting material] value, is adjusted. The molar ratio can be adjusted by additionally supplying or withdrawing the starting material and/or hydrogen fluoride to or from the distillation column or other lines. [The number of moles of the hydrogen fluoride]/[the number of moles of the unreacted starting material] value is preferably adjusted to 5 to 230, more preferably 5 to 18.58, and particularly preferably 5 to 17. Further, the lower limit of [the number of moles of the hydrogen fluoride]/[the number of moles of the unreacted starting material] value is also preferably 10. The phrase "the pressure in the distillation column" as used here refers to a gauge pressure (i.e., pressure in relation to atmospheric pressure taken as 0) unless otherwise stated.

Another method for performing distillation under conditions in which liquid-liquid separation does not occur is, for example, a method in which the pressure in the distillation column is adjusted. For instance, the pressure in the distillation column can be adjusted by changing the temperature in the distillation column. Alternatively, the pressure in the distillation column can be adjusted by supplying the reaction mixture, starting material, hydrogen fluoride, and/or others, such as inert gas, to the distillation column or discharging the reaction mixture, starting material, hydrogen fluoride, and/or others, such as inert gas.

The occurrence of liquid-liquid separation in the distillation column can be determined from the liquid density of a stream withdrawn from the distillation column. Specifically, when liquid-liquid separation occurs at a portion of the distillation column from which the second stream is withdrawn, an organic layer (layer containing the starting material), which is a lower layer, is mainly withdrawn from the distillation column; therefore, the liquid density of the stream withdrawn from the distillation column is higher than when liquid-liquid separation does not occur, and becomes a value close to the liquid density of the starting material alone. When such a change in the liquid density is observed, it can be determined that liquid-liquid separation has occurred.

The pressure in the distillation column is not particularly limited. For example, the pressure in the distillation column may be 0 to 1 MPa.

As described above, distillation is performed under conditions in which the unreacted starting material and the hydrogen fluoride do not undergo liquid-liquid separation, and the second stream containing the unreacted starting material and the unreacted hydrogen fluoride is withdrawn from the distillation column. If the second stream is withdrawn from the still of the distillation column, i.e., the bottom of the distillation column (the bottom of the column), the still can be heated to 33°C or more, which makes liquid-liquid separation much less likely to occur. The temperature of the still is particularly preferably 50°C or more. In particular, when the pressure in the liquid-liquid separation curve of 1233xf-HF is 0.34 MPa (absolute pressure), the temperature of the still is preferably 33°C or more, and more preferably 50°C or more.

In step (c), the second stream withdrawn from the distillation column is fed to the reactor where the reaction of step (a) is performed. Thus, the unreacted starting material and the unreacted hydrogen fluoride can be recycled to the reaction of step (a). For example, the second stream can be fed to the reactor while pressure is applied with a pump, a compressor, or the like.

More specifically, conditions for the distillation can be set as follows.

First, the pressure in the distillation column where the distillation of step (b) is performed is defined as $Y_1$ (MPa; gauge pressure), the molar ratio of the hydrogen fluoride to the unreacted starting material (i.e., [the number of moles of the hydrogen fluoride]/[the number of moles of the unreacted starting material]) is defined as $X_1$, and the relationship between $X_1$ and $Y_1$ is plotted. For example, the relationship between $X_1$ and $Y_1$ (10 to 50 points) is plotted in the $Y_1$ range of 0 to 1 MPa and the $X_1$ range of 5 to 25. By plotting in such a manner, an X-Y curve is drawn, and a relational expression that expresses $Y_1$ as a function of $X_1$ is calculated from this curve. Automated calculation software may be used to calculate this relational expression.

If the relational expression is expressed as $f(X_1)$, as long as the relationship of $Y_1 \geq f(X_1)$ is satisfied, the distillation can be performed under conditions in which liquid-liquid separation does not occur. Thus, the molar ratio $X_1$ and the pressure in the distillation column $Y_1$ can be adjusted so as to satisfy this relationship to perform the distillation. The molar ratio $X_1$ is preferably adjusted to 5 to 230, more preferably 5 to 18.58, and particularly preferably 5 to 17. Further, the lower limit of $X_1$ is also preferably 10.

As described above, withdrawing the second stream without the occurrence of liquid-liquid separation in the distillation of step (b) prevents a high concentration of the unreacted starting material in the second stream, resulting in the suppression of deactivation of the catalyst used in the reaction of step (a). If liquid-liquid separation into two phases occurs, the lower phase is the unreacted starting material, which has a greater specific gravity. Thus, if the second stream in a state in which liquid-liquid separation occurs is withdrawn, the unreacted starting material is recycled in a high concentration to the reactor. If the amount of such organic matter (unreacted starting material) is too large in the reaction system, the catalyst tends to be covered with the organic matter, leading to deactivation of the catalyst. However, withdrawing the second stream without the occurrence of liquid-liquid separation in step (b) prevents a high concentration of the unreacted starting material from being fed to the reactor as described above, making the deactivation of the catalyst unlikely to occur. This enables the chloropropene intermediate to be stably produced for a long period of time.

Method for Producing 2,3,3,3-tetrafluoropropene

2,3,3,3-tetrafluoropropene (which hereinafter may be referred to as "HFO-1234yf" or "1234yf") is produced using a chloropropene represented by formula (II): $CX_3CCl=CH_2$, wherein at least one X is F and the other or others are Cl or F, and each X may be the same or different (i.e., which corresponds to 2-chloro-3,3,3-trifluoropropene, which is the chloropropene intermediate of the first stage) as a raw material.

Hereinafter, the stage of producing HFO-1234yf from the chloropropene intermediate is referred to as "second stage."

The second stage comprises the following steps (d) to (f) :

(d) reacting the chloropropene of formula (II) with hydrogen fluoride in the presence of a catalyst;
(e) subjecting the reaction mixture obtained in step (d) to distillation to separate the mixture into a first stream comprising 2,3,3,3-tetrafluoropropene as a main component and a second stream comprising the unreacted hydrogen fluoride and organic matter containing the unreacted chloropropene represented by formula (II) as main components; and
(f) recycling the second stream separated in step (e) to the reaction of step (d).

In particular, in the present invention, the distillation of step (e) is performed under conditions in which the unreacted chloropropene intermediate and the unreacted hydrogen fluoride do not undergo liquid-liquid separation at a portion of the distillation column from which the second stream is withdrawn.

In step (d), the chloropropene intermediate is reacted with hydrogen fluoride to obtain the desired HFO-1234yf (2,3,3,3-tetrafluoropropene).

The method for reacting the chloropropene intermediate with hydrogen fluoride in the presence of a catalyst is not particularly limited. Examples of specific embodiments of the method include a method in which a catalyst is placed in a tubular flow reactor, and the chloropropene intermediate and hydrogen fluoride used as raw materials are introduced into the reactor.

The chloropropene intermediate can be reacted with hydrogen fluoride in a gas phase. The chloropropene intermediate and hydrogen fluoride are brought into contact with each other in a gaseous state in the reaction temperature region described below. When the chloropropene intermediate is liquid at an ordinary temperature and ordinary pressure, the chloropropene intermediate may be evaporated using an evaporator and supplied to a reactor where the reaction of step (d) is performed.

Hydrogen fluoride may be supplied to the reactor, for example, in the same manner as in step (a), and the method is not particularly limited. The amount of hydrogen fluoride supplied is generally about 1 to 100 moles, and preferably about 5 to 50 moles, per mole of the chloropropene intermediate. By setting the amount with such a range, the conversion of the chloropropene intermediate can be maintained within a desirable range. An amount of hydrogen fluoride of 10 moles or more per mole of the chloropropene intermediate is particularly preferable because the deactivation of the catalyst can be suppressed.

The molar ratio of hydrogen fluoride and the chloropropene intermediate to be supplied to the reactor can be adjusted by the amounts of hydrogen fluoride and the chloropropene intermediate supplied to the reactor. Thus, for a stream for supplying the major raw materials to the reactor and the second stream, the flow rates of hydrogen fluoride and the starting material can be adjusted by additionally supplying these materials or withdrawing them from the reactor.

Hydrogen fluoride and the chloropropene intermediate may be supplied to the reactor together with gas that is inert to the raw materials and the catalyst, such as nitrogen, helium, or argon. The concentration of inert gas may be about 0 to 80 mol% based on the total amount of the raw materials including the chloropropene intermediate and hydrogen fluoride introduced into the reactor and the inert gas, plus, when added, oxygen gas described later.

The chloropropene intermediate may be supplied to the reactor of step (d) together with oxygen. In this case, the amount of oxygen supplied may be about 0.1 to 50 mol% based on the total amount of the raw materials and oxygen, plus, when added, inert gas.

As a catalyst, known materials that have been used for this reaction can be used, and the type of catalyst is not particularly limited. For example, the catalysts mentioned above as usable in step (a) can also be used in step (d).

The reactor is preferably a tubular reactor. The method for bringing a material into contact with the catalyst is preferably a method using a fixed layer. The reactor is preferably made of a material resistant to the corrosive action of hydrogen fluoride, such as Hastelloy (registered trademark), Inconel (registered trademark), or Monel (registered trademark).

In the reaction of step (d), the reaction temperature is not particularly limited and is generally preferably about 200°C to 550°C. When the temperature is in this range, excellent conversion of the chloropropene intermediate into the desired product is exhibited, and the production of by-products caused by decomposition of the raw materials can be suppressed. The reaction temperature is more preferably about 300°C to 450°C.

The pressure during the reaction of step (d) is not particularly limited, and the reaction may be performed under reduced pressure, ordinary pressure, or increased pressure. Although the reaction may be generally carried out at a pressure near atmospheric pressure (0.1 MPa), it can also proceed smoothly under reduced pressure of less than 0.1 MPa. Furthermore, the reaction may be performed under increased pressure within a range in which the raw materials do not liquefy.

There is no limitation on the reaction time. For example, the contact time represented by W/F0, i.e., the ratio of the amount of packed catalyst W(g) to the total flow rate F0 (a flow rate at 0°C and 0.1 MPa: cc/sec) of gas components supplied to the reaction system, is preferably about 0.1 to 90 g·sec/cc, and more preferably about 1 to 50 g·sec/cc. Here, the total flow rate of gas components refers to the total flow rate of the chloropropene intermediate and hydrogen fluoride, and, when used, inert gas, oxygen, etc.

In step (d), the product obtained in the first stage may be supplied to the reactor of step (d) as is, but is preferably supplied to the reactor of step (d) after removing hydrogen chloride contained in the product. Due to this, the effects of reducing energy loss caused by handling hydrogen chloride that is unnecessary in step (d) and improving the selectivity of the desired HFO-1234yf can be expected. The method for removing hydrogen chloride from the product obtained in the first stage is not particularly limited. For example, hydrogen chloride can be easily removed as a column top fraction by the distillation of step (e) after step (d).

A reaction mixture containing the desired HFO-1234yf as a main component and also containing the unreacted chloropropene intermediate and the unreacted hydrogen fluoride is obtained by performing step (d) described above. The reaction mixture also contains hydrogen chloride (HCl) produced as a by-product in the reaction in addition to HFO-1234yf etc.

In step (e), the reaction mixture obtained in step (d) is subjected to distillation. The distillation can be performed using a commonly used distillation column or the like. By this distillation, the mixture can be separated into a first stream comprising HFO-1234yf as a main component and a second stream comprising the unreacted hydrogen fluoride and organic matter containing at least the unreacted chloropropene intermediate as main components. The portion of the distillation column from which the second stream is withdrawn may be the still of the distillation column or a middle portion of the distillation column. The "organic matter containing at least the unreacted chloropropene intermediate" in the second stream contains generally 90% or more chloropropene intermediate and less than 10% of other organic compounds. Examples of other organic compounds include 1233zd (1-chloro-3,3,3-trifluoropropene), 1223xd (1,2-dichloro-3,3,3-trifluoropropene), and the like.

The first stream contains the desired HFO-1234yf as a main component and also contains other components such as hydrogen chloride and 1,1,1,2,2-pentafluoropropane (hereinafter referred to as "245cb") produced as a by-product in the reaction of step (d). The obtained HFO-1234yf can be further subjected to a crude purification step and a fine purification step to yield a final product. Specific methods for the crude purification step and the fine purification step are not particularly limited. For example, water washing, dehydration (drying), distillation, liquid-liquid separation or other means can be applied to the steps.

The distillation described above is performed under conditions in which the unreacted hydrogen fluoride and organic matter containing the unreacted chloropropene intermediate that form the second stream do not undergo liquid-liquid separation.

Examples of the method for performing distillation under conditions in which liquid-liquid separation does not occur include a method in which the molar ratio of the hydrogen fluoride relative to 1 mole of the organic matter in the distillation column, i.e., [the number of moles of the hydrogen fluoride]/[the number of moles of the organic matter] value, more specifically [the number of moles of the hydrogen fluoride]/[the number of moles of the unreacted chloropropene intermediate] value is adjusted. The molar ratio can be adjusted by additionally supplying or withdrawing the chloropropene intermediate and/or hydrogen fluoride to or from the distillation column or other lines. [The number of moles of the hydrogen fluoride]/[the number of moles of the unreacted chloropropene intermediate] value is preferably adjusted to 5 to 230, more preferably 5 to 18.58, and particularly preferably 5 to 17. Further, the lower limit of [the number of moles of the hydrogen fluoride]/[the number of moles of the unreacted chloropropene intermediate] value is also preferably 10. The pressure in the distillation column is not particularly limited and may be, for example, 0 to 1 MPa.

Another method for performing distillation under conditions in which liquid-liquid separation does not occur is, for example, a method in which the pressure in the distillation column is adjusted. For instance, the pressure in the distillation column can be adjusted by changing the temperature in the distillation column. Alternatively, the pressure in the distillation column can be adjusted by supplying the reaction mixture, hydrogen fluoride, and/or others, such as inert gas, to the distillation column or discharging the reaction mixture, hydrogen fluoride, and/or others, such as inert gas.

The occurrence of liquid-liquid separation in the distillation column can be determined from the liquid density of a stream withdrawn from the still of the distillation column. Specifically, when liquid-liquid separation occurs at a portion of the distillation column from which the second stream is withdrawn, an organic layer (a layer containing the starting material), which is a lower layer, is mainly withdrawn from the distillation column; therefore, the liquid density of the stream withdrawn from the distillation column is higher than when liquid-liquid separation does not occur, and becomes a value close to the liquid density of the starting material alone. When such a change in the liquid density is observed, it can be determined that liquid-liquid separation has occurred.

As described above, distillation is performed under conditions in which the chloropropene intermediate and the hydrogen fluoride do not undergo liquid-liquid separation, and the second stream containing the chloropropene intermediate and the hydrogen fluoride is withdrawn from the distillation column. If the second stream is withdrawn from the still of the distillation column, i.e., the bottom of the distillation column (the bottom of the column), the still may be, for example, heated to 33°C or more since liquid-liquid separation is much less likely to occur. The temperature of the still is particularly preferably 50°C or more.

More specifically, conditions for the distillation can be set as follows.

First, the pressure in the distillation column in which the distillation of step (e) is performed is defined as $Y_2$ (MPa; gauge pressure), the molar ratio of the hydrogen fluoride relative to 1 mole of the organic matter (i.e., [the number of moles of the hydrogen fluoride]/[the number of moles of the organic matter]) is defined as $X_2$, and the relationship between $X_2$ and $Y_2$ is plotted. The organic matter here means the chloropropene intermediate. The relationship between $X_2$ and $Y_2$ (10 to 50 points) is plotted in the same ranges as in the distillation in the first stage. By plotting in such a manner, an X-Y curve is drawn, from which a relational expression that expresses $Y_2$ as a function of $X_2$ is calculated. Automated calculation software may be used to calculate this relational expression.

If the relational expression is expressed as $f(X_2)$, as long as the relationship of $Y_2 \geq f(X_2)$ is satisfied, the distillation can be performed under conditions in which liquid-liquid separation does not occur. Thus, the molar ratio $X_2$ and the pressure in the distillation column $Y_2$ can be adjusted so as to satisfy this relationship to perform the distillation. The molar ratio $X_2$ is preferably 5 to 17. The molar ratio $X_2$ is preferably adjusted to 5 to 230, more preferably 5 to 18.58, and particularly preferably 5 to 17. The lower limit of the molar ratio $X_2$ is also preferably 10.

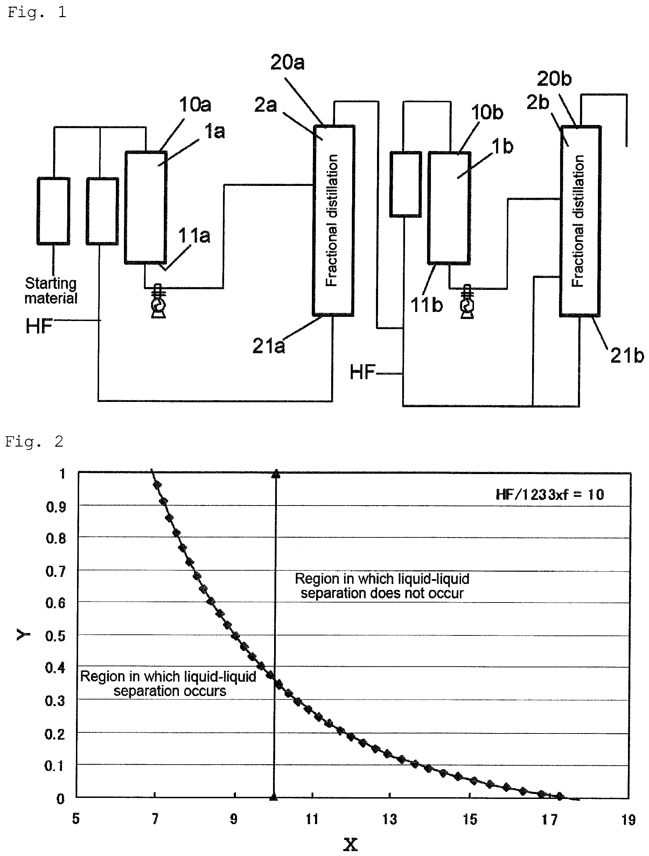

Fig. 2 is a curve (liquid-liquid separation curve) derived from the relationship between $Y_2$ and $X_2$ by using the above method when the chloropropene intermediate is 2-chloro-3,3,3-trifluoropropene. If the chloropropene intermediate is 2-chloro-3,3,3-trifluoropropene, the relationship between $Y_2$ and $X_2$ is derived from the liquid-liquid separation curve shown in Fig. 2 to obtain the following equation (1).

$$Y \geq -0.000027546483936X^5 + 0.001787977446337X^4 - 0.046613778365698X^3$$
$$+0.617838436698327X^2 - 4.260415044902270X + 12.671429439720600 \quad (1)$$

When the chloropropene intermediate is 2-chloro-3,3,3-trifluoropropene, liquid-liquid separation of the hydrogen fluoride and the organic matter containing the chloropropene intermediate can be prevented by determining $X_2$ and $Y_2$ and performing distillation in such a manner that the relationship of equation (1) is satisfied. In particular, deactivation of the catalyst is further suppressed in the region in Fig. 2 in which $X \geq 10$ and $Y_2 \geq f(X_2)$.

In step (f), the second stream withdrawn from the distillation column is fed to the reactor where the reaction of step (d) is performed. Thus, the chloropropene intermediate and the hydrogen fluoride can be recycled to the reaction of step (d). For example, the second stream can be fed to the reactor while pressure is applied with a pump, a compressor, or the like.

As described above, withdrawing the second stream without the occurrence of liquid-liquid separation in the distillation of step (e) prevents a high concentration of the chloropropene intermediate in the second stream, resulting in suppression of the deactivation of the catalyst used in the reaction of step (d). If liquid-liquid separation into two phases occurs, the lower phase is the chloropropene intermediate, which has a greater specific gravity. Thus, if the second stream in a state in which liquid-liquid separation occurs is withdrawn, the chloropropene intermediate is fed in a high concentration to the reactor. If the amount of the organic matter, such as the chloropropene intermediate, is too large in the reaction system, the catalyst tends to be covered with the organic matter, leading to the deactivation of the catalyst. However, withdrawing the second stream without the occurrence of liquid-liquid separation in step (e) prevents a high concentration of the chloropropene intermediate from being fed to the reactor, making the deactivation of the catalyst unlikely to occur. This enables HFO-1234yf to be stably produced for a long period of time.

HFO-1234yf can also be produced by using the first stage and second stage in combination. Specifically, the chloropropene intermediate of formula (II) can be produced from a starting material containing the compound of formula (Ia) and/or the compound of formula (Ib) in the first stage, and then, HFO-1234yf can be produced, in the second stage, from the chloropropene intermediate obtained in the first stage.

Also, when HFO-1234yf is produced by using the first stage and second stage in combination as described above, the first stage comprises step (a), step (b), and step (c) mentioned above, and the second stage comprises step (d), step (e), and step (f) mentioned above.

The distillation of step (b) is performed under conditions in which the unreacted starting material and the unreacted hydrogen fluoride do not undergo liquid-liquid separation, or the distillation of step (e) is performed under conditions in which the unreacted hydrogen fluoride and the organic matter containing the unreacted chloropropene intermediate do not undergo liquid-liquid separation. Alternatively, the distillation of step (b) is performed under conditions in which the unreacted starting material and the unreacted hydrogen fluoride do not undergo liquid-liquid separation, and the distillation of step (e) is performed under conditions in which the unreacted hydrogen fluoride and the organic matter containing the unreacted chloropropene intermediate do not undergo liquid-liquid separation. This makes the deactivation of the catalyst unlikely to occur in the first stage and/or the second stage, enabling the chloropropene intermediate of the first stage and HFO-1234yf of the second stage to be stably produced for a long period of time.

Fig. 1 is a schematic flow diagram illustrating an example of the flow for the production of HFO-1234yf. In the production flow of Fig. 1, HFO-1234yf is produced in a production line comprising a first reactor 1a, a second reactor 1b, a first distillation column 2a, a second distillation column 2b, and the like. The reaction of step (a) in the first stage can be performed in the first reactor 1a, and the reaction of step (d) in the second stage can be performed in the second reactor 1b.

More specifically, the starting material is supplied from the inlet side 10a of the first reactor 1a together with hydrogen fluoride to allow a reaction to proceed in the presence of a catalyst. Subsequently, the reaction products are withdrawn from the outlet side 11a of the first reactor 1a. The reaction mixture containing a chloropropene intermediate as a product, the unreacted starting material chloropropane, and the unreacted hydrogen fluoride is supplied to the first distillation column 2a where step (b) is performed.

In the first distillation column 2a, the chloropropene intermediate as a product is withdrawn as a first stream from the top 20a of the first distillation column 2a and is supplied to the second reactor 1b. The unreacted starting material chloropropane and the unreacted hydrogen fluoride are withdrawn as a second stream from the still 21a of the first distillation column 2a and are supplied to the first reactor 1a to be reused for the reaction of step (a). As described above, the distillation is performed under conditions in which the unreacted starting material and the unreacted hydrogen fluoride do not undergo liquid-liquid separation.

The chloropropene intermediate supplied to the second reactor 1b is reacted with hydrogen fluoride in the presence of a catalyst. The hydrogen fluoride used here may be separately supplied to the second reactor 1b. Subsequently, the reaction products are withdrawn from the outlet side 11b of the second reactor 1b. The reaction mixture containing the desired HFO-1234yf as a main component and also containing the unreacted chloropropene intermediate and the unreacted hydrogen fluoride is supplied to the second distillation column 2b where step (e) is performed.

HFO-1234yf supplied to the second distillation column 2b is withdrawn from the top 20b of the second distillation column 2b by distillation. The hydrogen chloride and the like are then removed by purification or like treatment. The chloropropene intermediate and the unreacted hydrogen fluoride are withdrawn from the still 21b of the second distillation column 2b and are supplied to the second reactor 1b to be reused for the reaction of step (d). As described above, the distillation is performed under conditions in which the unreacted chloropropene intermediate and the unreacted hydrogen fluoride do not undergo liquid-liquid separation. The production flow mentioned above is an example for producing HFO-1234yf, and HFO-1234yf may be produced in a production line other than that shown in Fig. 1.

Examples

Examples are given below to illustrate the present invention in more detail; however, the present invention is not limited to these Examples.

Example 1

HFO-1234yf was produced according to the production flow shown in Fig. 1. Cylinder-shaped reactors made of Hastelloy C22 were used. Cylinder-shaped packed columns made of Hastelloy C22 were used as distillation columns. The packing used was CMRNo2.5, the column diameter was 500A, and the packed length was 10000 mm $\times$ 2.

240db (1,1,1,2,3-pentachloropropane) was used as a starting material in step (a) of the first stage. A mixed gas of 240db and hydrogen fluoride was continuously supplied to a first reactor 1a at a flow rate of 7,000 $m^3$/hr (in terms of standard conditions for gas). The internal temperature of the first reactor 1a was 300°C, and the pressure was 0.75 MPa (gauge pressure). Further, in the reaction, the molar ratio of hydrogen fluoride to 240db was 20. To the first reactor 1a, 24.8 t of a Cr oxide catalyst ($Cr_2O_3$) was supplied as a catalyst in advance.

After the reaction, the reaction products were withdrawn from the first reactor 1a, fed to a first distillation column 2a, and subjected to distillation. The unreacted 240db and the unreacted hydrogen fluoride were withdrawn from the still of the first distillation column 2a and fed to the first reactor 1a again to be reused as raw materials for the reaction. From the top of the first distillation column 2a, 1233xf (2-chloro-3,3,3-trifluoropropene) as a product was withdrawn and fed to a second reactor 1b where the reaction of the next second stage was performed. In the reaction, the conversion of 240db to 1233xf was 50 to 99%.

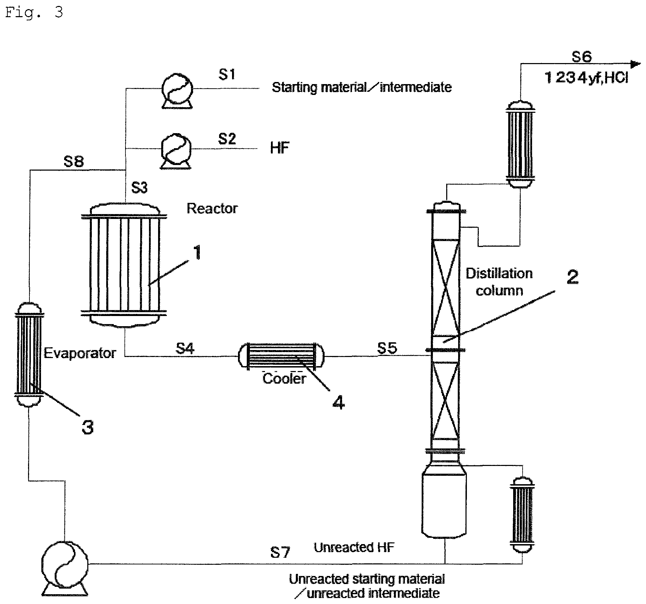

Fig. 3 is a schematic flow diagram illustrating the process from reaction to distillation in more detail. The materials of the evaporator 3 and the cooler 4 shown in Fig. 3 were Hastelloy C22 in this Example.

1233xf was fed through the S1 line of Fig. 3 from the first distillation column 2a, hydrogen fluoride was supplied from the S2 line, and these two came together in the S3 line. The mixed gas of 1233xf and hydrogen fluoride was continuously supplied to a reactor 1 through the S3 line at a flow rate of 21,000 $m^3$/hr (in terms of standard conditions for gas). In the reactor 1, 1233xf was reacted with hydrogen fluoride in the presence of 49.6 t of a Cr oxide catalyst ($Cr_2O_3$) used as a catalyst. The internal temperature of the reactor 1 was 365°C, and the pressure was 0.75 MPa (gauge pressure). Further, in this reaction, the molar ratio of hydrogen fluoride to 1233xf was 10. After the reaction, the obtained reaction mixture was fed from the reactor 1 to a distillation column 2. In the reaction in the reactor 1, 1,1,1,2,2-pentafluoropropane (245cb) was produced as a by-product.

The distillation in the distillation column 2 was performed under the following conditions: a column top temperature of 33°C, a column bottom temperature of 70°C, a pressure of 0.75 MPa, and a reflux ratio of 3.4. The reflux ratio here means the molar flow ratio of reflux liquid to distillate (reflux liquid/distillate). A mixture containing HCl and the desired HFO-1234yf was withdrawn from the top of the column, and a mixture containing the unreacted hydrogen fluoride and the unreacted 1233xf was withdrawn from the still (the bottom of the column). For the distillation, the molar ratio of hydrogen fluoride and 1233xf flowed in the S1, S2, and S7 lines of Fig. 3, the flow rates, and the pressure were adjusted (the molar ratio of hydrogen fluoride and 1233xf was about 12) so that hydrogen fluoride and 1233xf were maintained in the state of a single phase without liquid-liquid separation in the distillation column 2 (i.e., under conditions that satisfy the above equation (1)). The mixture containing the unreacted hydrogen fluoride and the unreacted 1233xf withdrawn from the still was recycled in the second reactor.

Table 1 shows the flow rates of gases in each of the S1 to S8 lines in Fig. 3.

Table 1

| Example 1 | S1 | S2 | S3 | S4 | S5 | S6 | S7 | S8 |
|---|---|---|---|---|---|---|---|---|
| HF [kmol/hr] | 0 | 0.34 | 12 | 11.76 | 11.76 | 0.10 | 11.66 | 11.66 |
| HCl [kmol/hr] | 0 | 0 | 0 | 0.24 | 0.24 | 0.24 | 0 | 0 |
| 1234yf [kmol/hr] | 0 | 0 | 0 | 0.24 | 0.24 | 0.24 | 0 | 0 |
| 1233xf [kmol/hr] | 0.25 | 0 | 1.2 | 0.96 | 0.96 | 0.01 | 0.95 | 0.95 |
| 245cb [kmol/hr] | 0 | 0 | 0 | 0.04 | 0.04 | 0.04 | 0 | 0 |
| Total flow rate [kmol/hr] | 0 | 0.34 | 13.2 | 13.24 | 13.24 | 0.63 | 12.61 | 12.61 |

Example 2

1233xf was produced from 240db according to the production flow shown in Fig. 3. First, 240db was fed through the S1 line, hydrogen fluoride was fed through the S2 line, and these two came together in the S3 line. The mixed gas of 240db and hydrogen fluoride was continuously supplied to a reactor 1 at a flow rate of 7,000 m$^3$/hr (in terms of standard conditions for gas). The internal temperature of the reactor 1 was 300°C, and the pressure was 0.75 MPa (gauge pressure). Further, in this reaction, the molar ratio of hydrogen fluoride to 240db was 20. To the reactor 1, 24.8 t of a Cr oxide catalyst ($Cr_2O_3$) was supplied as a catalyst in advance. After the reaction, the reaction mixture was withdrawn from the reactor 1, fed to a distillation column 2, and subjected to distillation. The same reactor, distillation column, evaporator, and cooler as used in Example 1 were used for the reactor 1, distillation column 2, evaporator 3, and cooler 4.

The distillation in the distillation column 2 was performed under the following conditions: a column top temperature of 6.63°C, a column bottom temperature of 93.6°C, a pressure of 0.75 MPa, and a reflux ratio of 5. A mixture containing 1233xf was withdrawn from the top of the column, and a mixture containing the unreacted hydrogen fluoride and the unreacted 240db was withdrawn from the still (the bottom of the column). The unreacted 240db and unreacted hydrogen fluoride withdrawn from the still were fed to the reactor 1 again to be reused as raw materials for the reaction. For the distillation, the molar ratio of hydrogen fluoride and 240db flowed in the S1, S2, and S7 lines of Fig. 3, the flow rates, and the pressure were adjusted (the molar ratio of hydrogen fluoride and 240db was about 229) so that hydrogen fluoride and 240db were maintained in the state of a single phase without liquid-liquid separation in the distillation column 2.

Table 2 shows the flow rates of gases in each of the S1 to S8 lines in Fig. 3.

Table 2

| Example 2 | S1 | S2 | S3 | S4 | S5 | S6 | S7 | S8 |
|---|---|---|---|---|---|---|---|---|
| HCl [kmol/hr] | 0 | 0 | 0 | 4.09 | 4.09 | 4.09 | 0 | 0 |
| HF [kmol/hr] | 0 | 8.42 | 40.49 | 37.42 | 37.42 | 5.35 | 32.07 | 32.07 |
| 1233xf [kmol/hr] | 0 | 0 | 0.14 | 1.16 | 1.16 | 1.02 | 0.14 | 0.14 |
| 240db [kmol/hr] | 1.02 | 0 | 2.04 | 1.02 | 1.02 | 0 | 1.02 | 1.02 |
| Total flow rate [kmol/hr] | 1.02 | 8.42 | 42.67 | 43.69 | 42.69 | 10.46 | 33.23 | 33.23 |

Example 3

1233xf was produced from 1230xa (1,1,2,3-tetrachloropropene) according to the production flow shown in Fig. 3. First, 1230xa was fed through the S1 line, hydrogen fluoride was fed through the S2 line, and these two came together in the S3 line. The mixed gas of 1230xa and hydrogen fluoride was continuously supplied to a reactor 1 at a flow rate of 7,000 m³/hr (in terms of standard conditions for gas). The internal temperature of the reactor 1 was 300°C, and the pressure was 0.75 MPa. Further, in this reaction, the molar ratio of hydrogen fluoride to 1230xa was 20. To the reactor 1, 24.8 t of a Cr oxide catalyst (Cr₂O₃) was supplied as a catalyst in advance. After the reaction, the reaction mixture was withdrawn from the reactor 1, fed to a distillation column 2, and subjected to distillation. The unreacted 1230xa and the unreacted hydrogen fluoride were withdrawn from the still of the distillation column 2 and fed to the reactor 1 again to be reused as raw materials for the reaction. The same reactor, distillation column, evaporator, and cooler as used in Example 1 were used for the reactor 1, distillation column 2, evaporator 3, and cooler 4.
The distillation in the distillation column 2 was performed under the following conditions: a column top temperature of -13.2°C, a column bottom temperature of 89.6°C, a pressure of 0.75 MPa, and a reflux ratio of 4. A mixture containing 1233xf was withdrawn from the top of the column, and a mixture containing the unreacted hydrogen fluoride and the unreacted 1230xa was withdrawn from the still (the bottom of the column). For the distillation, the molar ratio of hydrogen fluoride and 1230xa flown in the S1, S2, and S7 lines of Fig. 3, the flow rates, and the pressure were adjusted (the molar ratio of hydrogen fluoride and 1230xa was about 220) so that hydrogen fluoride and 1230xa were maintained in the state of a single phase without liquid-liquid separation in the distillation column 2.
Table 3 shows the flow rates of gases in each of the S1 to S8 lines in Fig. 3.

Table 3

| Example 3 | S1 | S2 | S3 | S4 | S5 | S6 | S7 | S7 |
|---|---|---|---|---|---|---|---|---|
| HCl [kmol/hr] | 0 | 0 | 0 | 4.97 | 4.97 | 4.97 | 0 | 0 |
| HF [kmol/hr] | 0 | 6.76 | 33.18 | 28.21 | 28.21 | 1.79 | 26.42 | 26.42 |
| 1233xf [kmol/hr] | 0 | 0 | 0.12 | 1.77 | 1.77 | 1.66 | 0.12 | 0.12 |
| 1230xa [kmol/hr] | 1.66 | 0 | 1.67 | 0.01 | 0.01 | 0 | 0.01 | 0.01 |
| Total flow rate [kmol/hr] | 1.66 | 6.76 | 34.96 | 34.96 | 34.96 | 8.41 | 26.55 | 26.55 |

Example 4

According to the production flow shown in Fig. 3, 1233xf was produced from 243db (2,3-dichloro-1,1,1-trifluoropropane). First, 243db was fed through the S1 line, hydrogen fluoride was fed through the S2 line, and these two came together in the S3 line. The mixed gas of 243db and hydrogen fluoride was continuously supplied to a reactor 1 at a flow rate of 7,000 m³/hr (in terms of standard conditions for gas). The internal temperature of the reactor 1 was 300°C, and the pressure was 0.75 MPa. Further, in this reaction, the molar ratio of hydrogen fluoride to 243db was 20. To the reactor 1, 24.8 t of a Cr oxide catalyst (Cr₂O₃) was supplied as a catalyst in advance. After the reaction, the reaction mixture was withdrawn from the reactor 1, fed to a distillation column 2, and subjected to distillation. The same reactor, distillation column, evaporator, and cooler as used in Example 1 were used for the reactor 1, distillation column 2, evaporator 3, and cooler 4.
The distillation in the distillation column 2 was performed under the following conditions: a column top temperature of 1.24°C, a column bottom temperature of 81.1°C, a pressure of 0.75 MPa, and a reflux ratio of 5. A mixture containing 1233xf was withdrawn from the top of the column, and a mixture containing the unreacted hydrogen fluoride and the unreacted 243db was withdrawn from the still (the bottom of the column). The unreacted 243db and unreacted hydrogen fluoride withdrawn from the still were fed to the reactor 1 again to be reused as raw materials for the reaction. For the distillation, the molar ratio of hydrogen fluoride and 243db flown in the S1, S2, and S7 lines of Fig. 3, the flow rates, and the pressure were adjusted (the molar ratio of hydrogen fluoride and 243db was about 18) so that hydrogen fluoride and 243db were maintained in the state of a single phase without liquid-liquid separation in the distillation column 2.
Table 4 shows the flow rates of gases in each of the S1 to S8 lines in Fig. 3.

Table 4

| Example 4 | S1 | S2 | S3 | S4 | S5 | S6 | S7 | S8 |
|---|---|---|---|---|---|---|---|---|
| HCl [kmol/hr] | 0 | 0 | 0 | 1.95 | 1.95 | 1.95 | 0 | 0 |

(continued)

| Example 4 | S1 | S2 | S3 | S4 | S5 | S6 | S7 | S8 |
|---|---|---|---|---|---|---|---|---|
| HF [kmol/hr] | 0 | 2.36 | 33.38 | 33.06 | 33.06 | 1.04 | 32.02 | 32.02 |
| 1234yf [kmol/hr] | 0 | 0 | 0 | 0.32 | 0.32 | 0.32 | 0 | 0 |
| 245cb [kmol/hr] | 0 | 0 | 0.19 | 0.19 | 0.19 | 0 | 0.19 | 0.19 |
| 1233xf [kmol/hr] | 0 | 0 | 1.57 | 2.88 | 2.88 | 1.31 | 1.57 | 1.57 |
| 243db [kmol/hr] | 1.63 | 0 | 1.64 | 0.01 | 0.01 | 0 | 0.01 | 0.01 |
| Total flow rate [kmol/hr] | 1.63 | 2.36 | 36.78 | 38.41 | 38.41 | 4.62 | 33.79 | 33.79 |

Example 5

According to the production flow shown in Fig. 3, 1233xf was produced from 244bb (2-chloro-1,1,1,2-tetrafluoropropane). First, 244bb was fed through the S1 line, and a mixed gas of this 244bb and hydrogen fluoride was continuously supplied to a reactor 1 at a flow rate of 7,000 m³/hr (in terms of standard conditions for gas). In this reaction, hydrogen fluoride was released from 244bb used as a raw material; therefore, hydrogen fluoride was not supplied from the S2 line. The internal temperature of the reactor 1 was 300°C, and the pressure was 0.75 MPa. Further, in this reaction, the molar ratio of hydrogen fluoride to 244bb was 20. To the reactor 1, 24.8 t was supplied as a catalyst in advance. After the reaction, the reaction mixture was withdrawn from the reactor 1, fed to a distillation column 2, and subjected to distillation. The same reactor, distillation column, evaporator, and cooler as used in Example 1 were used for the reactor 1, distillation column 2, evaporator 3, and cooler 4.

The distillation in the distillation column 2 was performed under the following conditions: a column top temperature of 6.63°C, a column bottom temperature of 93.6°C, a pressure of 0.75 MPa, and a reflux ratio of 4. A mixture containing 1233xf was withdrawn from the top of the column, and a mixture containing the unreacted hydrogen fluoride and the unreacted 244bb was withdrawn from the still (the bottom of the column). The unreacted 244bb and unreacted hydrogen fluoride withdrawn from the still were fed again to the reactor 1 to be used as raw materials for the reaction. For the distillation, the molar ratio of hydrogen fluoride and 244bb flowed in the S1, S2, and S7 lines of Fig. 3, the flow rates, and the pressure were adjusted (the molar ratio of hydrogen fluoride and 244bb was about 12) so that hydrogen fluoride and 244bb were maintained in the state of a single phase without liquid-liquid separation in the distillation column 2.

Table 5 shows the flow rates of gases in each of the S1 to S8 lines in Fig. 3.

Table 5

| Example 5 | S1 | S2 | S3 | S4 | S5 | S6 | S7 | S8 |
|---|---|---|---|---|---|---|---|---|
| HCl [kmol/hr] | 0 | 0 | 0 | 1.19 | 1.19 | 1.19 | 0 | 0 |
| HF [kmol/hr] | 0 | 0 | 8.10 | 12.17 | 12.17 | 4.07 | 8.10 | 8.10 |
| 1234yf [kmol/hr] | 0 | 0 | 0 | 1.19 | 1.19 | 1.19 | 0 | 0 |
| 1233xf [kmol/hr] | 0 | 0 | 0.27 | 4.34 | 4.34 | 4.07 | 0.27 | 0.27 |
| 244bb [kmol/hr] | 5.25 | 0 | 5.65 | 0.40 | 0.40 | 0 | 0.40 | 0.40 |
| Total flow rate [kmol/hr] | 5.25 | 0 | 14.02 | 19.27 | 19.27 | 10.50 | 8.77 | 8.77 |

Comparative Example

Under the same conditions as in Example 1, 1233xf was produced from 240db by the reaction of the first stage. Subsequently, the obtained 1233xf was withdrawn from the top of a first distillation column 2a and fed to a second reactor 1b where the reaction of the second stage was performed. More specifically, according to the production flow shown in Fig. 3, 1233xf was fed through the S1 line, hydrogen fluoride was supplied through the S2 line, and these two came together in the S3 line. The mixed gas of 1233xf and hydrogen fluoride was continuously supplied to a reactor 1 through the S3 line at a flow rate of 21,000 m³/hr (in terms of standard conditions for gas). In the reactor 1, 1233xf was reacted with hydrogen fluoride in the presence of 49.6 t of a Cr oxide catalyst ($Cr_2O_3$) used as a catalyst. The internal temperature of the reactor 1 was 365°C, and the pressure was 0.1 MPa (gauge pressure). Further, in this reaction, the molar ratio of hydrogen fluoride to 1233xf was 10, and W/F0 was 10. After the reaction, the obtained reaction mixture was fed from the reactor 1 to a distillation column 2.

The distillation in the distillation column 2 was performed under the following conditions: a column top temperature of 33°C, a column bottom temperature of 70°C, a pressure of 0.1 MPa, and a reflux ratio of 3.4. A mixture containing HCl and the desired HFO-1234yf was withdrawn from the top of the column, and a mixture containing the unreacted hydrogen fluoride and the unreacted 1233xf was withdrawn from the still. For the distillation, the molar ratio of hydrogen fluoride and 1233xf flowed in the S1, S2, and S7 lines of Fig. 3, the flow rates, and the pressure were adjusted so that hydrogen fluoride and 1233xf underwent liquid-liquid separation in the distillation column 2 (i.e., under conditions that do not satisfy the above equation (1)). The mixture containing the unreacted hydrogen fluoride and unreacted 1233xf withdrawn from the still was recycled in the second reactor.

Deactivation of Catalyst

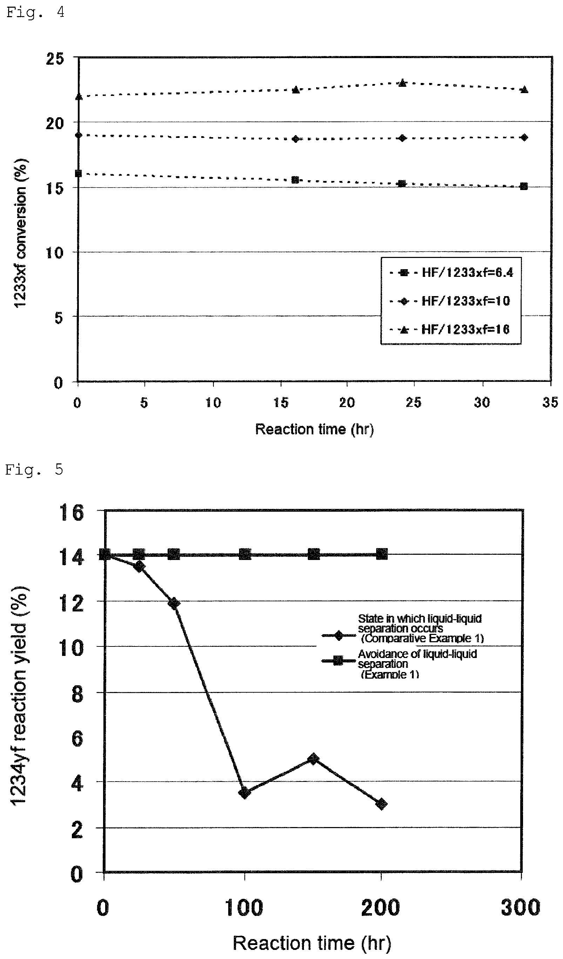

Fig. 4 is a graph plotting the relationship between the conversion of 1233xf to 1234yf and the reaction time in Example 1. Fig. 4 also shows results obtained in the case in which the molar ratio of hydrogen fluoride to 1233xf in the reaction of the second stage of Example 1 was changed to 6.4 and in the case in which the molar ratio of hydrogen fluoride to 1233xf in the reaction of the second stage of Example 1 was changed to 16 (the pressure in the distillation column in both cases was 0.75 MPa). This graph indicates that when the molar ratio was 6.4, which is a condition that does not satisfy equation (1) described above (see also Fig. 2), the catalyst was deactivated, resulting in low conversion, and the conversion further decreased with the reaction time. In contrast, when the molar ratio was 10 or 16, which are conditions that satisfy equation (1) (see also Fig. 2), deactivation of the catalyst was suppressed, resulting in high conversion, and the degree of a decrease in the conversion was suppressed in spite of the elapse of the reaction time compared with the case in which the molar ratio was 6.4.

Fig. 5 is a graph plotting the relationship between the reaction time and the reaction yield of HFO-1234yf in the reaction of the second stage of Example 1 and plotting the relationship between the reaction time and the reaction yield of HFO-1234yf in the reaction of the Comparative Example. In the Comparative Example, the reaction yield decreased with the elapse of the reaction time. In contrast, in Example 1, a decrease in the reaction yield was not observed even 200 hours after the start of the reaction. Since the distillation was performed under conditions in which hydrogen fluoride and 1233xf underwent liquid-liquid separation in the Comparative Example, a large amount of organic matter was returned to the reactor when reflux was performed. Thus, the catalyst was deactivated by the action of the organic matter, causing a decrease in the reaction yield. In contrast, in Example 1, the distillation was performed under conditions in which hydrogen fluoride and 1233xf did not undergo liquid-liquid separation (i.e., under conditions that satisfy the above equation (1); therefore, even though reflux was performed, deactivation of the catalyst was unlikely to occur, enabling the reaction to be stably performed for a long period of time.

Description of Reference Numerals

1     Reactor
1a    First reactor
1b    Second reactor
2     Distillation column
2a    First distillation column
2b    Second distillation column
3     Evaporator
4     Cooler

**Claims**

1. A method for producing 2,3,3,3-tetrafluoropropene from 2-chloro-3,3,3-trifluoropropene,
   the method comprising the following steps (d) to (f):

   (d) reacting 2-chloro-3,3,3-trifluoropropene with hydrogen fluoride in the presence of a catalyst;
   (e) subjecting the reaction mixture obtained in step (d) to distillation to separate the mixture into a first stream comprising 2,3,3,3-tetrafluoropropene as a main component and a second stream comprising unreacted hydrogen fluoride and organic matter containing unreacted 2-chloro-3,3,3-trifluoropropene as main components; and

(f) recycling the second stream separated in step (e) above to the reaction of step (d),

wherein the distillation of step (e) is performed under conditions that satisfy the relationship of the following equation (1):

$$Y_2 \geq -0.000027546483936X_2^5 + 0.001787977446337X_2^4$$
$$-0.046613778365698X_2^3 + 0.617838436698327X_2^2$$
$$-4.260415044902270X_2 + 12.671429439720600 \quad (1),$$

wherein $Y_2$ is the pressure in a distillation column where the distillation of step (e) is performed, and $X_2$ is the molar ratio of the hydrogen fluoride to the organic matter in the distillation column.

2. The production method according to claim 1, wherein the molar ratio $X_2$ is 10 or more, and the pressure $Y_2$ is 0 MPa or more but 1 MPa or less.

3. A method for producing 2,3,3,3-tetrafluoropropene, the method comprising:

a first stage of obtaining 2-chloro-3,3,3-trifluoropropene from a starting material containing a chloropropane represented by formula (Ia): $CX_3CClYCH_2Y$, wherein X is Cl or F, each X may be the same or different, Y is H, F, or Cl, and each Y may be the same or different, and/or a chloropropene represented by formula (Ib): $CY_3CCl=CZ_2$, wherein Y is H or Cl, each Y may be the same or different, Z is H, F, or Cl, and each Z may be the same or different; and
a second stage of obtaining 2,3,3,3-tetrafluoropropene from the 2-chloro-3,3,3-trifluoropropene,
the first stage comprising the following steps (a) to (c);

(a) reacting the starting material with hydrogen fluoride in the presence of a catalyst;
(b) subjecting the reaction mixture obtained in step (a) to distillation to separate the mixture into a first stream comprising the 2-chloro-3,3,3-trifluoropropene as a main component and a second stream comprising an unreacted starting material and unreacted hydrogen fluoride as main components;
(c) recycling the second stream separated in step (b) to the reaction of step (a),

the second stage comprising the following steps (d) to (f):

(d) reacting the 2-chloro-3,3,3-trifluoropropene with hydrogen fluoride in the presence of a catalyst;
(e) subjecting the reaction mixture obtained in step (d) to distillation to separate the mixture into a first stream comprising 2,3,3,3-tetrafluoropropene as a main component and a second stream comprising unreacted hydrogen fluoride and organic matter containing unreacted 2-chloro-3,3,3-trifluoropropene as main components; and
(f) recycling the second stream separated in step (e) to the reaction of step (d),

wherein the distillation of step (e) is performed under conditions that satisfy the relationship of the following equation (1):

$$Y_2 \geq -0.000027546483936X_2^5 + 0.001787977446337X_2^4$$
$$-0.046613778365698X_2^3 + 0.617838436698327X_2^2$$
$$-4.260415044902270X_2 + 12.671429439720600 \quad (1),$$

wherein $Y_2$ is the pressure in a distillation column where the distillation of step (e) is performed, and $X_2$ is the molar ratio of the hydrogen fluoride to the organic matter in the distillation column.

4. The production method according to claim 3, wherein the distillation of step (b) is performed under conditions in which the unreacted starting material and the unreacted hydrogen fluoride do not undergo liquid-liquid separation.

5. The production method according to claim 3 or 4, wherein the molar ratio $X_2$ is 10 or more, and the pressure $Y_2$ is

0 MPa or more but 1 MPa or less.

6. The production method according to any one of claims 3 to 5, wherein the starting material is 1,1,1,2,3-pentachloropropane, the amount of the hydrogen fluoride supplied to a reactor is 15 moles or more per mole of the starting material supplied to the reactor, and the pressure in the distillation column where the distillation of step (b) is performed is 0 MPa or more but 1 MPa or less.

Fig. 1

Fig. 2

Fig. 3

Fig. 4

Fig. 5

## INTERNATIONAL SEARCH REPORT

| International application No. |
| --- |
| PCT/JP2016/087804 |

### A. CLASSIFICATION OF SUBJECT MATTER

*C07C17/383*(2006.01)i, *C07C17/25*(2006.01)i, *C07C21/18*(2006.01)i, *C07B61/00* (2006.01)n

According to International Patent Classification (IPC) or to both national classification and IPC

### B. FIELDS SEARCHED

Minimum documentation searched (classification system followed by classification symbols)
C07C17/383, C07C17/25, C07C21/18, C07B61/00

Documentation searched other than minimum documentation to the extent that such documents are included in the fields searched
| | | | |
| --- | --- | --- | --- |
| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2014-500858 A (Arkema France),<br>16 January 2014 (16.01.2014),<br>claims 1, 2; paragraphs [0038] to [0040],<br>[0066] to [0069]; examples 1, 7; fig. 1, 2, 4<br>& US 2013/0267740 A1<br>claims 1, 2; paragraphs [0069] to [0071],<br>[0099] to [0101]; fig. 1, 2, 4<br>& WO 2012/052797 A1 & EP 2630108 A<br>& CN 103180275 A & MX 2013004415 A<br>& RU 2013123286 A | 1-6 |
| X | US 2011/0160497 A1 (Arkema France),<br>30 June 2011 (30.06.2011),<br>fig. 2, 3; paragraphs [0041] to [0044];<br>examples 1, 7; claims 36 to 38, 43 to 44<br>& WO 2011/077192 A1 & EP 2516366 A<br>& CN 102686542 A | 1-6 |

| ☒ Further documents are listed in the continuation of Box C. | ☐ See patent family annex. |
| --- | --- |

<table>
<tr><td>*</td><td>Special categories of cited documents:</td><td>"T"</td><td>later document published after the international filing date or priority date and not in conflict with the application but cited to understand the principle or theory underlying the invention</td></tr>
<tr><td>"A"</td><td>document defining the general state of the art which is not considered to be of particular relevance</td><td></td><td></td></tr>
<tr><td>"E"</td><td>earlier application or patent but published on or after the international filing date</td><td>"X"</td><td>document of particular relevance; the claimed invention cannot be considered novel or cannot be considered to involve an inventive step when the document is taken alone</td></tr>
<tr><td>"L"</td><td>document which may throw doubts on priority claim(s) or which is cited to establish the publication date of another citation or other special reason (as specified)</td><td>"Y"</td><td>document of particular relevance; the claimed invention cannot be considered to involve an inventive step when the document is combined with one or more other such documents, such combination being obvious to a person skilled in the art</td></tr>
<tr><td>"O"</td><td>document referring to an oral disclosure, use, exhibition or other means</td><td></td><td></td></tr>
<tr><td>"P"</td><td>document published prior to the international filing date but later than the priority date claimed</td><td>"&"</td><td>document member of the same patent family</td></tr>
</table>

| Date of the actual completion of the international search | Date of mailing of the international search report |
| --- | --- |
| 16 January 2017 (16.01.17) | 24 January 2017 (24.01.17) |

| Name and mailing address of the ISA/<br>Japan Patent Office<br>3-4-3,Kasumigaseki,Chiyoda-ku,<br>Tokyo 100-8915,Japan | Authorized officer<br><br><br>Telephone No. |
| --- | --- |

Form PCT/ISA/210 (second sheet) (January 2015)

**INTERNATIONAL SEARCH REPORT**

International application No.

PCT/JP2016/087804

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2013-510075 A  (Daikin Industries, Ltd.), 21 March 2013 (21.03.2013), paragraphs [0062] to [0063] & US 2012/0222448 A1    & WO 2011/059078 A1 & EP 2499110 A          & CN 102596868 A & KR 10-2012-0070604 A | 1-6 |
| A | JP 2011-513227 A  (E.I. Du Pont de Nemours & Co.), 28 April 2011 (28.04.2011), examples 3, 6 & US 2012/0261252 A1    & WO 2009/105512 A1 & EP 2247561 A1         & CA 2713086 A & MX 2010009080 A        & CN 101952230 A & KR 10-2010-0138933 A  & RU 2010138783 A & ES 2463670 T           & BR PI0906014 A & KR 10-2016-0120804 A | 1-6 |
| A | JP 2015-522521 A  (Daikin Industries, Ltd.), 06 August 2015 (06.08.2015), examples & US 2015/0203421 A1    & WO 2014/025065 A1 & EP 2882704 A          & CN 104520259 A | 1-6 |
| P,X | WO 2016/009774 A1  (Daikin Industries, Ltd.), 21 January 2016 (21.01.2016), entire text & JP 2016-20319 A | 1-6 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2996555 A

- WO 2008002499 A